# EUROPÄISCHE PATENTSCHRIFT

(11) **EP 3 868 201 B1**
(45) Veröffentlichungstag und Bekanntmachung des Hinweises auf die Patenterteilung: **05.01.2022**
(21) Anmeldenummer: 20020077.2
(22) Anmeldetag: 20.02.2020
(51) Int. Cl.: A01K 67/033

(54) **INSEKTEN-MASTWANNE**
INSECT REARING BOX
BAC D'ÉLEVAGE D'INSECTES

(43) Veröffentlichungstag der Anmeldung: 25.08.2021
(73) Patentinhaber: madebymade GmbH, 04523 Pegau (DE)
(72) Erfinder: Herrmann, Klemens, 67307 Göllheim (DE); Finck, Jonas, 04564 Böhlen (DE); Hempel, Kai Uwe, 04275 Leipzig (DE); Weinreis, Yannik Andreas, 04207 Leipzig (DE)
(74) Vertreter: Köhler, Tobias

(56) Entgegenhaltungen:
- CN-A- 103 478 084
- JP-A- 2001 300 478
- KR-B1- 102 024 635
- KR-B1- 102 078 152

## Beschreibung

Die Erfindung betrifft eine Mastwanne zum Einsatz bei der weltweiten, hygienischen, sicheren und effektiven Zucht von flugunfähigen Insekten.

Die Erschließung alternativer Proteinquellen ist von hoher Bedeutung, da die Produktion konventioneller Proteinquellen an der Kapazitätsgrenze ist und große Umweltschäden verursacht. Zusätzlich steigt stetig der Bedarf an Protein für die menschliche Ernährung aber auch für die Ernährung von Nutztleren. Insekten sind eine sehr kllmafreundliche alternative Protelnquelle. Die Herausforderung ist Systeme zu entwickeln, die eine Industrielle Zucht ermöglichen.

In der KR 102 024 635 B1 wird die Bereitstellung eines Behälters zur Behandlung organischer Abfälle vorgeschlagen, der in der Lage ist, Insektenlarven von organischen Abfällen, die biologisch mit Insekten behandelt werden, ohne Zeit- und Arbeitsaufwand zu trennen. Nachteilig bei dieser Lösung ist, dass eine Flucht der Insekten nicht verhindert werden kann.

In der WO201829597 wird eine technologische Anlage zum Aufziehen und / oder Züchten von flugunfählgen Insekten und / oder Larvenformen von Insekten beschrieben, welche dadurch gekennzeichnet ist, dass sie mindestens eine Ebene umfasst, wobei die Ebene ein autonomes Förderband (11) mit seitlichen Seitenwänden (6a, 6b ) auf beiden Seiten entlang der Bewegungsrichtung des Förderbandes (11) platziert, wobei die oberen Kanten wenigstens einmal nach innen gekrümmt sind, wobei der Biegewinkel (a) der Kante jeder Seitenwand nicht weniger als 30 ° beträgt, vorzugsweise aus 30 ° bis 90 °, und Querbügel (12), die sich im allgemeinen senkrecht zu den seitlichen Seitenwänden (6a, 6b) erstrecken und das Band des Förderers (11) tragen, die die gegenüberliegenden profilierten seitlichen Seitenwände (6a, 6b) verbinden die profilierten seitlichen Seltenwände (6a, 6b) sind ebenfalls Strukturelemente, die Längskanten des Bandes des Förderers (11) tragen, so dass sowohl das Förderband (11) als auch die seitlichen Seitenwände (6a, 6b) ein Wannenprofil bilden und der Druck der Masse der Insekten, auf der Stirnseite des Förderbandes (11) und gegebenenfalls zusätzlich der Futter- und / oder Exkremente von Insekten drücken an den Längskanten des Förderbandes (11) gegen die profilierten seitlichen Seitenwände (6a, 6b);
wobei sowohl die Querbügel (12) als auch die profilierten Seitenwände (6a, 6b) aus einem Material mit effizienten Wärmeleiteigenschaften bestehen.
Aus der US20150164109 ist ein Verfahren zur Herstellung von Lebensmittelinsekten bekannt, welches die folgenden Verfahrensschritte umfasst: Bereitstellen von Lebensmittelabfällen;
Entfernen von physischen Gefahren, falls vorhanden, aus dem Lebensmittelabfall;
Zerkleinern der Lebensmittelabfälle und Behandeln der Lebensmittelabfälle, die wirksam sind, um biologische Lebensmittelrisiken zu verringern und dadurch Insektenfutter bereitzustellen;
Anpflanzen von Insekten auf dem Insektenfutter; und Ernte der Insekten.

Die CA2965088 beschreibt eine Einheit (1), eine Insektenzuchtanlage und ein kombiniertes System zur Insektenproduktion zur Züchtung von Insekten, dadurch gekennzeichnet, dass sie mindestens ein geschlossenes Gehäuse (2), das Folgendes umfasst: - mindestens eine obere Kammer (3), die Insekten enthalten soll, wobei die oben genannte obere Kammer (3) mindestens einen Trichter (6) umfasst; - mindestens ein unteres Abteil (5), das zur Aufnahme des in der oberen Kammer (3) enthaltenen Insekts bestimmt ist; - die oben genannten Kompartimente (3) und Unterkörper (5) sind abnehmbare und trennbare Hüllen (2), unabhängig voneinander; - mindestens ein Trennelement (4) der vorgenannten Kompartimente überlegen (3) und minderwertig (5), wobei das vorgenannte Element (4) Poren einschließt; ein Überwachungssystem der Umgebungsbedingungen des Gehäuses.

Nachteilig dabei ist jedoch, dass diese Lösungen sehr komplex ausgeführt sind und solche Anlagen regelmäßig auch immobil sind.

Ebenfalls ist von Nachteil, dass die beschriebenen Lösungen für Mastwannen nur unter großem Aufwand zu reinigen sind und somit eine leichte und gute Automatisierung nur bedingt möglich ist.

Aufgabe der Erfindung ist es, die bekannten Nachteile des Standes der Technik zu beheben und somit einen Vorschlag zu unterbreiten, mit welchem auch bei Einsatz von organischen, aggressiven Inputstoffen (Fruchtsäuren etc.) eine langlebige, ökologische und leichter zu reinigende Ausführung vorgeschlagen wird, welche ebenfalls die hygienischen Anforderungen erfüllt.

Gelöst wird diese Aufgabe durch eine Mastwanne 1 nach Anspruch 1, mit an ihren Ecken angeordneten

Füßen 2 als Führung für einen auf diese Mastwanne 1 aufgesetzten und beweglich angeordneten Rahmen 3.

Diese erfindungsgemäße Lösung der Aufgabe soll anhand der Abbildungen 1 bis 4 näher erläutert werden.

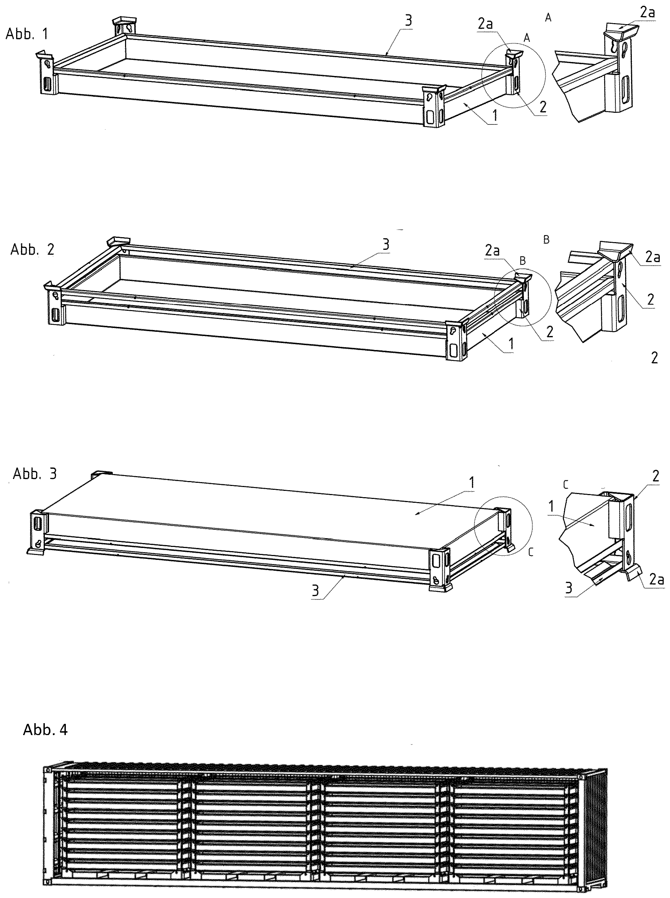

Dabei zeigt Abbildung 1 die erfindungsgemäße Mastwanne in normaler Lage, ergänzend zeigt das Detail A die Lage des Rahmens 3 in den Füßen 2.

Abbildung 2 zeigt die erfindungsgemäße Mastwanne 1 in normaler Lage mit angehobenem Rahmen 3, ergänzend zeigt das Detail B die Lage des Rahmens 3 in den Füßen 2.

Die Abbildung 3 zeigt die erfindungsgemäße Mastwanne 1 in umgedrehter Lage mit nach unten bewegten Rahmen 3, ergänzend zeigt das Detail C die Lage des Rahmens 3 in den Füßen 2.

Abbildung 4 zeigt eine beispielhafte Stapelung von Mastwannen 1 in einem 40 Fuß-Container zum Transport und Betrieb.

Nachfolgend soll die erfindungsgemäße Mastwanne 1 anhand der Abbildungen und eines Ausführungsbeispiels näher erläutert werden.

Es ist bei zukünftiger Massenzucht von Insekten durchaus relevant, ganze Zuchtanlagen oder aber auch nur die Mastwannen zu transportieren. Deshalb ist die erfindungsgemäße Mastwanne so gestaltet, dass sie optimal für die Zucht von flugunfähigen Insekten geeignet ist und gleichzeitig eine maximale Anzahl von Mastwannen 1 in einen Schiffscontainer eingelagert werden kann und auch innerhalb des Schiffscontainer betrieben werden kann.

Um dem Transportgedanken gemäß Abbildung 4 Rechnung zu tragen, wird in besonderer Ausgestaltung der erfindungsgemäßen Lösung vorgeschlagen, dass die Mastwannen 1 aus Edelstahl- Maxiplatten (3000mmx 1500mm) gefertigt werden können. Dieses Maß ist weltweit gängig und es fällt besonders wenig Verschnitt an.

Die Mastwanne 1 wird gebildet aus einem Boden und diesen umschließenden Seitenwänden und ist vorzugsweise rechteckig ausgeführt und an der Oberkante ihrer Seitenwände zweifach so nach Innen gekantet, dass ein der Größe der Mastwannen 1 entsprechender Rahmen 3, der durch sein Eigengewicht plan auf der Mastwanne 1 aufliegt und eine Flucht/Ausbrechen der flugunfähigen Insekten verhindert.

Der Rahmen 3 ist mit abgekanteten Innenseiten ausgestattet, damit eine Flucht der flugunfähigen Insekten verhindert wird.

Die zweifache Kantung nach Innen bewirkt ebenfalls, dass die flugunfähigen Insekten nicht über die Seitenwände der Mastwannen 1 aus der Mastwannen 1 hinausklettern können.

An den Ecken der Mastwanne 1 sind Füße 2 angeordnet. Diese Füße 2 sind vorzugsweise so ausgebildet, dass sie die Ecken der Mastwanne 1 und die des auf der Mastwanne 1 aufliegenden Rahmens 3 umgreifen. Dabei sind die Füße 2 so ausgeführt sind, dass sie höher sind als die Wanne 1. An den oberen Enden der Füße 2 sind Auflageflächen 2a angeordnet. Die Höhe der Füße 2 ist dabei so gewählt, dass diese eine vertikale Bewegung des Rahmens 2 in den durch die Füße 2 gebildete Führung definieren, welche in einer Richtung durch die Mastwanne 1 und in der anderen Richtung durch Auflageflächen 2a begrenzt wird.

Diese Auflageflächen 2a sind so gestaltet, dass bei Stapelung von mehreren Mastwannen 1 das untere Ende der Füße 2 einer obenstehenden Mastwanne 1 in den Auflageflächen 2a der untenstehenden Mastwanne 1 sicher aufgenommen und fixiert wird.

Der Vorteil der Ausgestaltung der erfindungsgemäßen Mastwanne 1 mit dem Rahmen 3 soll nachfolgend näher erläutert werden.

Zum Auskippen und Entleeren der Mastwanne 1 wird diese umgekippt. Die Auflageflächen 2a dienen dabei als Anschlag für den Rahmen 3, welcher beim Umkippen der Mastwanne 1 abfallen würde und durch die Ausgestaltung der Füße 2 in diesen geführt wird und in seiner Bewegung durch die Auflageflächen 2a begrenzt wird.

Wird die Mastwanne 1 nach Entleerung wieder in ihre Normalposition gebracht, fällt der Rahmen 3 ebenfalls wieder in seine Normalposition und liegt dann wieder plan auf der Oberkante der Mastwanne 1 auf.

Durch die Ausgestaltung der Füße 2 der erfindungsgemäßen Mastwanne 1 sind die Mastwannen 1 stapelfähig und lassen sich als Turm stellen. Dadurch sind keine weiteren Gestelle oder Halterungen beim Stapeln von Mastwannen 1 notwendig.

In einer besonderen Ausgestaltung der erfindungsgemäßen Mastwanne 1 sind deren Füße 2 so gestaltet, dass die Verwendung einer Transportsicherung realisierbar ist. Eine solche Transportsicherung kann beispielsweis darin bestehen, dass in den Füßen 2, wie in den Abbildungen 1 bis 3 beispielhaft gezeigt ist, Löcher so angeordnet sind, dass eine Transportsicherung unter Verwendung dieser Löcher für ein sicheres Bewegen von gestapelten Mastwannen schnell und einfach zu montieren ist.

Wie in Abbildung 4 gezeigt, ist es möglich, die erfindungsgemäßen Mastwannen 1 aufeinander so zu stapeln, dass dieser Stapel bzw. eine Zahl n solcher Stapel den Raum eines 40 Fuß- Container optimal ausnutzen kann.

Vorzugsweise werden dabei die Mastwannen auf einer Stapelhilfe aufgestapelt, welche den Maßen der Mastwannen 1 entspricht und an ihren Ecken Aufnahmen für die Füße 2 der Mastwannen 1 aufweist. In diese Aufnahmen können die Füße der untenstehenden Mastwanne 1 formschlüssig so eingesetzt werden, dass hiermit schon diese untere Mastwanne 1 lagesicher eingesetzt ist. Ebenfalls können diese Stapelhilfen mit Greifpunkten für beispielsweise Gabelstapler ausgestattet sein. Dadurch ist möglich, ganze Stapel von Mastwannen 1 einfach und mit einem Hub in die vorzugsweise verwendeten 40 Fuß- Container einzustapeln.

Der Aufbau der Mastwanne 1 wurde so gewählt, dass eine signifikante Menge an Insekten gezüchtet werden kann, allerdings Menge bzw. Populationsdichte optimal für die Entwicklung und Qualität der Insekten ist. Bei der Entwicklung der Größe Mastwanne 1 wurden auch die Punkte Produktionssicherheit und Hygiene berücksichtigt. Viele Insektenzüchter nehmen entweder offene große Betonbecken, die entweder nicht oder schwer zu reinigen sind. Dadurch erhöht sich die Seuchengefahr. Eine andere Möglichkeit sind viele kleine Kunststoffkisten, die einem größeren klimatisierenden Raum gelagert werden. Hier ist eine Krankheitsübertragung über die Luft ein hohes Risiko.

Vorzugsweise wird als Material für die Mastwannen 1 Edelstahl ausgewählt, da die Mastwannen 1 mit Insektenfutter gefüllt werden. Das Futter besteht u.a. aus Fruchtsäuren und anderen Stoffen, die Stahl, Legierungen und Kunststoffe angreifen. Außerdem sind im industriellen Prozess Kratzer und andere "Verletzungen" des Materials unausweichlich. Ein anderer Werkstoff z.B. eine Legierung wäre dadurch unbrauchbar. Edelstahl hat den weiteren Vorteil, dass es gut gereinigt und desinfiziert werden kann, was bei einer industriellen Tierzucht unbedingt betrachtet werden muss.

Die optimalen Bedingungen für die Zucht von Larven sind je nach Entwicklungsstadium unterschiedlich und hängen von verschiedenen Parametern ab. Außerdem zeigen manche Larven ein starkes Migrationsverhalten. In einer industriellen Zuchtanlage ist es daher notwendig dieses Verhalten zu steuern. Mit der erfindungsgemäßen Lösung wurde eine sehr innovative und effiziente Variante entwickelt, die mit einem Rahmen 3 funktioniert, der durch die seine Konstruktion automatisch in die notwendige Position fällt und durch abgekantete Innenseiten die Flucht von flugunfähigen Insekten verhindert. Diese Entwicklung ist ersetzt bisherige aufwendige klimagesteuerte Technik mit hohem Energieaufwand.

## Patentansprüche

1. Mastwanne-Anordnung (1) zum Einsatz bei der weltweiten, hygienischen, sicheren und effektiven Zucht von flugunfähigen Insekten, umfassend eine Mastwanne, gebildet aus einem Boden und diesen umschließenden Seitenwänden, an den Ecken der Mastwanne (1) angeordneten Füßen (2) und einem beweglichen Rahmen, **dadurch gekennzeichnet, dass** die Seitenwände an ihrer Oberkante so nach Innen gekantet sind, dass der der Größe der Mastwanne (1) entsprechende Rahmen (3), der durch sein Eigengewicht plan auf der Mastwanne (1) aufllegt und mit abgekanteten Innenseiten ausgestattet ist, damit eine Flucht der flugunfähigen Insekten verhindert wird, wobei die Füße (2) so ausgeführt sind, dass sie höher sind als die Wanne (1) und
an den oberen Enden der Füße (2) Auflageflächen (2a) angeordnet sind, welche eine vertikale Bewegung des Rahmens (2) in den durch die Füße (2) gebildete Führung definieren und die Auflageflächen (2a) dabei so gestaltet sind, dass bei Stapelung von mehreren Mastwannen (1) das untere Ende der Füße (2) einer obenstehenden Mastwanne (1) in den Auflageflächen (2a) der untenstehenden Mastwanne (1) sicher aufgenommen und fixiert wird und dabei einen oberen Anschlag für den Rahmen (3) bilden.

2. Mastwanne-Anordnung (1) nach Anspruch 1, **dadurch gekennzeichnet, dass** die Füße (2) so ausgebildet sind, dass sie die Ecken der Mastwanne (1) und die des auf der Mastwanne (1) aufliegenden Rahmens (3) umgreifen.

3. Mastwanne-Anordnung (1) nach Anspruch 2, **dadurch gekennzeichnet, dass** in den Füßen (2) angeordneten Löchern angeordnet sind, durch welche eine Transportsicherung einzusetzen ist.

4. Mastwanne-Anordnung (1) nach Anspruch 1, **dadurch gekennzeichnet, dass** die Mastwanne (1) aus Edelstahl gefertigt ist.

5. Verfahren zum Stapeln von Mastwanne-Anordnungen (1) nach Anspruch 1, **dadurch gekennzeichnet, dass** Mastwanne-Anordnungen (1) auf einer Stapelhilfe aufzustapeln sind, welche den Maßen der Mastwanne-Anordnungen (1) entspricht und an ihren Ecken Aufnahmen für die Füße (2) der Mastwanne-Anordnungen (1) aufweist, in welche die Füße (2) der untenstehenden Mastwanne-Anordnungen (1) formschlüssig so eingesetzt werden, dass hiermit schon diese Mastwanne-Anordnung (1) lageslcher einzusetzen sind.

## Claims

1. The invention relates to a mast trough (1) arrangement for use in the worldwide, hygienic, safe and effective breeding of flightless insects, comprising a mast trough, formed from a floor and side walls surrounding it, feet (2) arranged at the corners of the mast trough (1) and a moving frame (3), the side walls being canted inwards at their upper edge that the frame (3) corresponding to the size of the mast trough (1), which rests flat on the mast trough (1) by its own weight and is equipped with bevelled inner sides so that flightless insects are prevented from escaping, the feet (2) are designed so that they are higher than the tub (1) and
at the upper ends of the feet (2) bearing surfaces (2a) are arranged, which define a vertical movement of the frame (2) in the guide formed by the feet (2) and the bearing surfaces (2a) are designed so that when stacked, the lower end of the feet (2) of an above mast trough (1) is securely received and fixed by several mast troughs (1) in the bearing surfaces (2a) of the mast trough below (1) and thereby form an upper stop for the frame (3).

2. A mast trough arrangement (1) according to Claim 1, **characterized in that** the feet (2) are so formed that they encompass the corners of the mast trough (1) and the frame (3) that rests flat on the mast trough (1).

3. A mast trough arrangement (1) according to Claim 2, **characterized by** holes having been inserted in the feet (2), these then being used for insertion of a transport lock.

4. A mast trough arrangement (1) according to Claim 1, **characterized by** the mast trough (1) being made of stainless steel.

5. A procedure for the stacking of mast trough arrangements (1) according to Claim 1, **characterized by** the mast trough arrangement (1) being stacked on a stacking aid that has the dimensions of the mast trough arrangements (1) and has supports for the feet at its corners for receiving the feet (2) of the mast trough arrangements (1), in which the feet (2) of the bottom mast trough arrangements (1) are so inserted that they are interlocked so that they are firmly in place.

## Revendications

1. Structure d'auge de mât (1) pour une utilisation dans l'élevage mondial, hygiénique, sûr et efficace des insectes incapables de voler, comprenant une auge de mât formée d'un fond et de parois latérales entourant le fond, des pieds (2) disposés aux coins de l'auge de mât (1 ) et un cadre mobile, **caractérisé en ce que** le bord supérieur des parois latérales est plié vers l'intérieur, de sorte que le cadre (3) correspondant à la dimension de l'auge de mât (1), qui repose à plat sur l'auge de mât (1) par son propre poids et est équipé de côtés intérieurs biseautés, de sorte à empêcher que les insectes incapables de voler ne s'échappent, les pieds (2) étant conçus de sorte qu'ils soient plus hauts que l'auge (1) et
et aux extrémités supérieures des pieds (2) sont disposées des surfaces d'appui (2a) qui définissent un mouvement vertical du cadre (2) dans le guidage formé par les pieds (2) et les surfaces d'appui (2a) sont conçues de telle sorte que, lorsque plusieurs auges de mât (1) sont empilées, l'extrémité des pieds (2) d'une auge de mât (1) se trouvant au-dessus est solidement supportée et fixée dans les surfaces d'appui (2a) de l'auge de mât (1) se trouvant au-dessous et forme ainsi une butée supérieure pour le cadre (3).

2. Structure d'auge de mât (1) selon la revendication 1, **caractérisé en ce que** les pieds (2) sont formés de manière à englober les coins de l'auge de mât (1) et ceux du cadre (3) reposant sur l'auge de mât (1).

3. Structure d'auge de mât (1) selon la revendication 2, **caractérisé en ce que** des trous sont ménagés dans les pieds (2), à travers lesquels une fixation de transport doit être insérée.

4. Structure d'auge de mât (1) selon la revendication 1, **caractérisé en ce que** l'auge de mât (1) est en acier inoxydable.

5. Procédé pour empiler des structures d'auge de mât (1) selon la revendication 1, **caractérisé en ce que** les structures d'auge de mât (1) doivent être empilées sur un dispositif d'empilage, qui correspond aux dimensions des structures d'auge de mât (1) et à leurs coins se trouvent des logements pour les pieds (2) des structures d'auge de mât (1), dans lesquels les pieds (2) des structures d'auge de mât (1) se trouvant au-dessous sont placés par adaptation de forme, de sorte que cette structure d'auge de mât (1) peut être utilisée de manière garantissant sa position.
